# EUROPEAN PATENT APPLICATION

(11) **EP 4 186 459 A1**
(43) Date of publication of application: **31.05.2023**
(21) Application number: 21210255.2
(22) Date of filing: 24.11.2021
(51) Int. Cl.: A61B 90/70

(54) **APPARATUS FOR THE WASHING AND DISINFECTION OF MEDICAL ITEMS**

(71) Applicant: Bonferraro S.p.A., 37060 Bonferraro (VR) (IT)
(72) Inventor: GOBBI, Ezio, Roncoferraro MN (IT); CALEFFI, Fausto, Viadana MN (IT); BENFATTI, Oscar, Isola della Scala VR (IT); ARTONI, Michele, Guastalla RE (IT)
(74) Representative: Concone, Emanuele

(57) **Abstract**

An apparatus for washing and disinfecting medical items, comprises a recycle tank (RT) and a boiler (DH) placed higher than the wash chamber (WC), a demineralised water supply line (DWL) for filling the boiler (DH) with demineralised water, a sump line (SL) branching off from the bottom sump (BS) of the wash chamber (WC) and equipped with valve means (EVS; EVR, EVW) and pumping means (SP) to load the recycle tank (RT) through a recycling line (RL) with the washing liquid discharged from the wash chamber (WC) at the end of the operation cycle, and a heat exchanger (HE) placed in the demineralised water supply line (DWL) which is located inside the recycle tank (RT) in order to pre-heat the demineralised water before loading it into the boiler (DH) by recovering heat from the washing liquid collected in the recycle tank (RT).

## Description

The present invention concerns washing and disinfection apparatuses (thermodisinfectors) for medical items such as robotic surgical instruments, cannulas for laparoscopy and the like, or glassware and in particular an apparatus provided with a heat exchanger to reduce the energy consumption and the duration of an operating cycle.

It is known that an apparatus for the washing and thermo-disinfection of medical items of this type traditionally includes a line for the supply of demineralised water to be used for rinsing and thermo-disinfection. In particular, robotic surgical instruments include micro-mechanisms with steel handling cables only a few tenths of a millimeter in diameter, therefore it is absolutely necessary to prevent solid impurities from being carried thereon during the washing, which could result in malfunction and damage.

Therefore, in order not to leave any kind of trace on the thermo-disinfected medical items and to prevent the deposit of potentially harmful substances dissolved in the mains water, demineralised water is used. This water is not only decalcified, as it occurs in simpler appliances equipped with a softener, but is treated in an external demineralisation device that strips it of practically all the mineral salts and substances it contains.

This rinsing water is loaded into the appliance through an electrovalve and collected in a special boiler to be pre-heated to 85°C before being introduced into the chamber for the final stages of rinsing and thermo-disinfection, when it is heated up to 93°C. At the end of the operating cycle, the water is discharged from the chamber and collected in a recycle tank to be reused in a pre-wash or first rinse phase of the following cycle, in order to reduce water consumption.

This well-known solution has the disadvantage of not exploiting the energy content of this very hot water from the thermo-disinfection phase, which requires about 46% of the entire energy consumption of an operating cycle. In fact, although the water saving for the above-mentioned pre-wash or first rinse phases of the following cycle is clear, such phases could be carried out also with cold water, and indeed in case of pre-wash a high water temperature could be contraindicated, as it could cause problems of fixation of proteins or other substances on the articles to be washed.

The purpose of the present invention is therefore to provide an apparatus which is free from such drawbacks. Said purpose is achieved by means of an apparatus comprising a heat exchanger placed in the demineralised water supply line between the inlet electrovalve and the boiler, said heat exchanger being located inside the aforementioned tank for recycling the water coming from the wash chamber at the end of the operating cycle. Other advantageous features of the present apparatus are specified in the dependent claims.

The main advantage of the present apparatus is to reduce the energy consumption of the boiler for the pre-heating of the demineralised water, since due to the recovery of heat from the water collected in the recycle tank the temperature of the demineralised water entering the boiler is not the usual 18-20°C but is already 46-48°C. Starting from this higher temperature, in tests carried out by the applicant the reduction in overall energy consumption for the entire operating cycle was 18-20%.

This also leads to a second significant advantage in terms of reducing the duration of the operating cycle, as heat recovery in the recycle tank takes approximately 3 minutes compared to the approximately 7 minutes that would be required by the boiler to supply the same amount of heat to the demineralised water.

Yet another advantage derives from the reduction in the temperature of the water collected in the recycle tank, which instead of being typically 90-92°C drops to 53-55°C due to the aforementioned heat transfer, making it more suitable for reuse in a pre-wash phase of the following operating cycle.

A further considerable advantage of this apparatus derives from the fact that it has a simple structure, which implies a reduced increase in cost compared to a traditional thermo-disinfector.

Further advantages and features of the apparatus according to the present invention will be evident to those skilled in the art from the following detailed description of an embodiment thereof with reference to the attached drawings wherein:
- Fig.1 represents a diagram of the main components of the apparatus; and
- Fig.2 represents a detail of a variant of the apparatus of Fig.1.

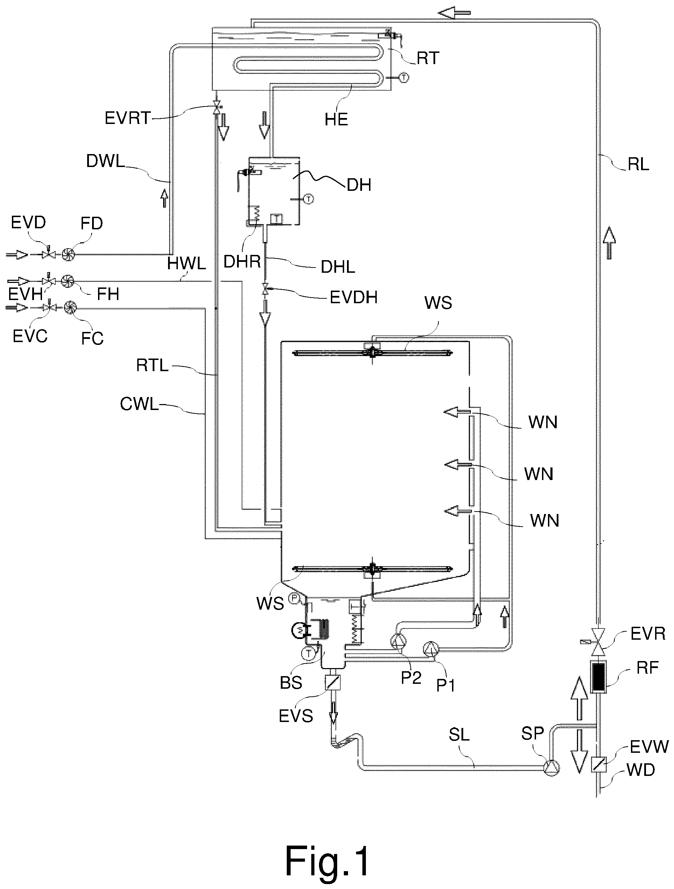

Referring to Fig.1, it can be seen that an apparatus according to the invention traditionally comprises a wash chamber WC with relative washing sprayers WS and washing nozzles WN fed by respective pumps P1 and P2 which draw the washing liquid from a bottom sump BS. At the end of the operating cycle, the water used for thermo-disinfection is discharged from sump BS through a sump line SL, by opening a relative electrovalve EVS and activating a relative pump SP.

The sump line SL terminates with a T-junction where on one side there is an electrovalve EVW that controls the flow of water towards a drain WD, and on the other side there is an electrovalve EVR (preferably preceded by a filter RF) that controls the flow of water towards a recycling line RL that leads to a recycle tank RT located higher up than the wash chamber WC. At the moment chosen in the following operating cycle, the water collected in the recycle tank RT is gravity fed into the wash chamber WC through a relative outflow line RTL, after opening a relative electrovalve EVRT that controls the outflow from the recycle tank RT.

As mentioned above, the apparatus also includes a demineralised water supply line DWL whose inlet is controlled by a relative electrovalve EVD preferably followed by a flowmeter FD, to measure the quantity of demineralised water that is loaded into a relative boiler DH. The latter is provided with at least one resistance DHR for preheating the demineralised water before it is gravity fed into the wash chamber WC through a relative outflow line DHL, after opening a relative electrovalve EVDH which controls the outflow from the boiler DH.

Finally, the apparatus also includes a cold water supply line CWL whose inlet is controlled by a relative electrovalve EVC preferably followed by a flowmeter FC, to measure the quantity of cold water that is directly loaded into the wash chamber WC. Optionally, the apparatus also comprises a hot water supply line HWL whose inlet is controlled by a relative electrovalve EVH preferably followed by a flowmeter FH, to measure the amount of hot water that is directly loaded into the wash chamber WC or into a relative boiler (not shown).

Note that the diagram in Fig.1 shows only the components necessary to understand the invention, but the apparatus includes numerous other components and lines which are necessary to perform other functions such as drying, steam condensation, addition of chemical additives and so on (i.e. pumps, filters, fans, resistances, thermostats, level sensors, electrovalves, etc.).

The innovative aspect of the present apparatus, as mentioned above, lies in the presence of a heat exchanger HE placed in the demineralised water supply line DWL between the inlet electrovalve EVD and the boiler DH, said heat exchanger HE being located inside the aforementioned recycle tank RT. In this way, it is possible to pre-heat the demineralised water in the heat exchanger HE thanks to the heat recovery from the water collected in the recycle tank RT, so that the temperature of the demineralised water entering the boiler DH is already increased by about 25-30°C and the resistance DHR has to work for less time to bring the water to the required temperature of 85°C.

This results in a reduction of energy consumption of the boiler DH in the order of 60%, and also a reduction of 4'/5' in the duration of the operating cycle. In this regard, it should be noted that it is also possible to prioritise the reduction of the energy load of the boiler DH while keeping its operating time unchanged by selecting a resistance DHR of lower power, which also has a lower cost, if the reduction of the operating cycle duration is not important.

By way of example, in the tests carried out by the applicant, an exchanger HE was used comprising a coil with a diameter of 1.6 cm and a length of about 12 m, which corresponds to a capacity of about 2.4 litres and an exchange surface of about 6000 cm². Obviously, other values are possible, preferably maintaining a capacity of at least 2 litres and an exchange surface of at least 5000 cm². The recycle tank RT typically has a capacity of 35-40 litres, and in any case is preferably large enough to contain all the water discharged from the wash chamber at the end of the operating cycle.

It is clear that the embodiment of the apparatus according to the invention described and illustrated above is only an example susceptible to numerous variations. In particular, the shape, size and arrangement of the heat exchanger HE and of the recycle tank RT may be varied as required, as long as the former is contained in the latter and both are arranged higher than the wash chamber WC.

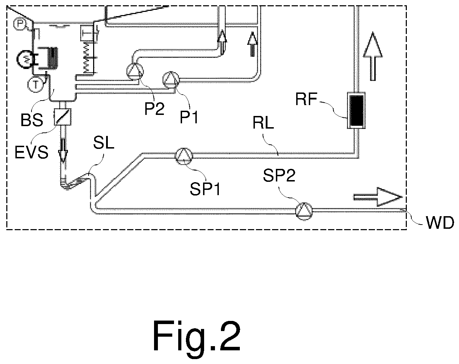

It should also be noted that the apparatus shown in Fig.1 may also vary in other aspects not relative to the heat exchanger HE, such as the number and arrangement of the washing components WS, WN in the wash chamber WC or the number of resistances in the boiler DH and sump BS. For example, as shown in the variant in Fig.2, the sump line SL instead of being equipped with only one pump SP and ending with a T-junction, where on one side is located the electrovalve EVW that controls the passage of the water towards the drain WD and on the other side is located the electrovalve EVR that controls the passage of the water towards the recycling line RL, could provide a Y-junction with two pumps SP1, SP2 each placed on one arm of the Y, where the first arm is connected to the recycling line RL and the second arm to the drain WD.

In this way, the water is conveyed to the drain WD or to the recycling line RL by simply activating one or the other pump, dispensing with the two electrovalves EVW, EVR. This solution simplifies the structure and operation of the apparatus, leading to a significant cost reduction, since the combination of the two pumps SP1, SP2 costs about one third of the cost of the combination of the SP pump plus the two electrovalves EVW, EVR.

## Claims

1. Apparatus for the washing and disinfection of medical items, comprising:
- a wash chamber (WC) equipped with a bottom sump (BS);
- a sump line (SL) branching off from said bottom sump (BS) and equipped with valve means (EVS; EVR, EVW) and pumping means (SP; SP1, SP2) for discharging washing liquid from said wash chamber (WC);
- a recycle tank (RT) located higher than the wash chamber (WC) and connected thereto through an outflow line (RTL) equipped with valve means (EVRT), as well as connected to said sump line (SL) through a recycling line (RL);
- a boiler (DH) equipped with at least one resistance (DHR) and located higher than the wash chamber (WC), to which it is connected through an outflow line (DHL) equipped with valve means (EVDH);
- a demineralised water supply line (DWL) which extends to said boiler (DH) and is equipped with inlet valve means (EVD) to control the filling of the boiler (DH) with demineralised water;
the washing liquid discharged from the wash chamber (WC) being directed to said recycle line (RL) or to a drain (WD) by selective actuation of said valve means (EVS; EVR, EVW) and of said pumping means (SP; SP1, SP2) of the sump line (SL), **characterized in that** it also comprises a heat exchanger (HE) arranged in said demineralised water supply line (DWL) between the inlet valve means (EVD) and the boiler (DH), said heat exchanger (HE) being located within said recycle tank (RT).

2. Apparatus according to claim 1, **characterized in that** the recycle tank (RT) is large enough to contain all the washing liquid discharged at the end of the operating cycle, preferably having a capacity of 35-40 litres.

3. Apparatus according to claim 1 or 2, **characterized in that** the exchanger (HE) has a capacity of at least 2 litres and an exchange surface of at least 5000 cm².

4. Apparatus according to any of the preceding claims, **characterized in that** the sump line (SL) ends in a T-junction wherein on one side is located an electrovalve (EVW) controlling the passage towards the drain (WD) and on the other side is located an electrovalve (EVR), preferably preceded by a filter (RF), controlling the passage towards the recycling line (RL), a pump (SP) being arranged on the sump line (SL) upstream of said T-junction.

5. Apparatus according to any of claims 1 to 3, **characterized in that** the sump line (SL) comprises a Y-connector with two pumps (SP1, SP2) each arranged on one arm of the Y, one of said arms being connected to the drain (WD) and the other being connected to the recycle line (RL), preferably through a filter (RF).
